# EUROPEAN PATENT SPECIFICATION

(11) **EP 4 574 813 B1**
(45) Date of publication and mention of the grant of the patent: **13.05.2026**
(21) Application number: 24808249.7
(22) Date of filing: 26.01.2024
(51) Int. Cl.: A61K 31/167, C07C 235/64, A61P 31/04, A61P 31/10, A61K 38/12

(54) **COMPOUND HAVING BROAD-SPECTRUM ANTIBACTERIAL ACTIVITY AND ANTIBACTERIAL COMPOSITION THEREOF**
VERBINDUNG MIT BREITSPEKTRUM ANTIBAKTERIELLER AKTIVITÄT UND ANTIBAKTERIELLE ZUSAMMENSETZUNG DAVON
COMPOSÉ AYANT UNE ACTIVITÉ ANTIBACTÉRIENNE À LARGE SPECTRE ET COMPOSITION ANTIBACTÉRIENNE ASSOCIÉE

(30) Priority: 23.10.2023 CN 202311372798
(43) Date of publication of application: 25.06.2025
(73) Proprietor: China Agricultural University, Beijing 100193 (CN)
(72) Inventor: SHEN, Jianzhong, Beijing 100193 (CN); ZHU, Kui, Beijing 100193 (CN); LI, Xiaoyu, Beijing 100193 (CN); ZHOU, Kaixiang, Beijing 100193 (CN); MAO, Changsi, Beijing 100193 (CN); CAI, Yawei, Beijing 100193 (CN); KANG, Jijun, Beijing 100193 (CN); WU, Congming, Beijing 100193 (CN); CAO, Xingyuan, Beijing 100193 (CN); LIU, Dejun, Beijing 100193 (CN)
(74) Representative: Huang, Liwei
(86) International application number: PCT/CN2024/074217
(87) International publication number: WO 2025/086494

(56) References cited:
- WO-A1-2020/172206
- CN-A- 109 475 515
- CN-A- 115 869 385
- CN-A- 115 919 830
- CN-A- 117 105 809
- CN-A- 117 105 810
- US-A1- 2022 143 005
- US-A1- 2022 356 147
- LAL JHAJAN ET AL: "Bio-evaluation of fluoro and trifluoromethyl-substituted salicylanilides against multidrug-resistant S. aureus", MEDICINAL CHEMISTRY RESEARCH, BIRKHAEUSER, BOSTON, US, vol. 30, no. 12, 27 October 2021 (2021-10-27), pages 2301 - 2315, XP038158252, ISSN: 1054-2523, [retrieved on 20211027], DOI: 10.1007/S00044-021-02808-4
- LI J ET AL: "Evaluation of colistin as an agent against multi-resistant Gram-negative bacteria", INTERNATIONAL JOURNAL OF ANTIMICROBIAL AGENTS, ELSEVIER, AMSTERDAM, NL, vol. 25, no. 1, 1 January 2005 (2005-01-01), pages 11 - 25, XP004693244, ISSN: 0924-8579, DOI: 10.1016/J.IJANTIMICAG.2004.10.001

## Description

### FIELD OF INVENTION

The present invention belongs to the field of antibacterial compound technology, and specifically relates to a compound, a composition, and the compound or the composition for medical use.

### BACKGROUND OF THE INVENTION

With the widespread use of antibiotics, resistance of pathogenic bacteria has become increasingly severe, and bacterial infections, especially those caused by multidrug-resistant bacteria, are facing a situation where no drugs are available. Clostridium perfringens has shown high resistance rates to many antibiotics, such as lincomycin (76.9%), doxycycline (69.2%), tilmicosin (65.4%), and tiamulin (50.0%), with some MIC values reaching 128 µg/mL or even higher. According to research, if left uncontrolled, the number of deaths caused by drug resistance is expected to reach 10 million by 2050. In response to the serious public health threat posed by bacterial resistance, the World Health Organization (WHO) and the US Food and Drug Administration (FDA) have successively released lists of bacteria in urgent need of novel antibiotics (WHO, 2017; FDA, 2017), demonstrating the urgency of developing novel antibiotics.

The development of "super antibiotics" capable of combating drug-resistant bacteria has become a rigid requirement for medical development. However, due to the limited variety of natural product structures and the decreasing frequency of discovery, the development of novel antibiotics from natural sources has become increasingly difficult. At the same time, due to the shorter treatment cycle of antibiotics compared to drugs for hypertension, diabetes, leukemia and other drugs, the development investment and income are relatively low, and the pharmaceutical companies have insufficient development incentives for novel antibiotics. In the past 50 years, only one new type of antibacterial drug from natural sources, daptomycin, has been put into clinical use. At present, the artificial design and modification of compounds that may have antibacterial potential is an important means of discovering novel antibacterial compounds, which has received widespread attention in recent years.

Benzoyl aniline derivatives are widely used, which have a backbone based on salicylic acid and are bound to aniline derivatives with aromatic rings through amide bonds. They have various biological activities such as weed control, insecticidal, antibacterial, and anticancer. Previous studies have modified the substituents of benzoyl aniline compounds while obtaining some compounds with antibacterial activity against Gram positive bacteria (Bakker et al, J Am Chem Soc, 2023, 145, 1136). Upon comparison, it can be found that the compounds provided by Bakker et al (2023) are mostly heterocyclic structures, and the 12 compounds provided by Bakker et al (2023) have the lowest MIC of 6.25 µg/mL against MRSA, which is not enough to meet the current practical needs. After searching, as of January 2023, there are two application patents based on benzoyl aniline as the parent nucleus structure. CN1958565A proposes a novel snail killing drug; CN102697760A proposes the application of niclosamide or its salts in the preparation of drugs for the prevention and treatment of pulmonary fibrosis. However, no such compounds have been reported as spectral antibacterial drugs, especially against bacteria and their composition.

LAL JHAJAN ET AL: "Bio-evaluation of fluoro and trifluoromethylsubstituted salicylanilides against multidrug-resistant S. aureus", MEDICINAL CHEMISTRY RESEARCH, BIRKHAEUSER, BOSTON, US, vol. 30, no. 12, 27 October 2021 (2021-10-27), pages 2301-2315, discloses many fluoro and trifluoromethylsubstituted salicylanilide derivatives in the prior art.

LI J ET AL: "Evaluation of colistin as an agent against multi-resistant Gram negative bacteria", INTERNATIONAL JOURNAL OF ANTIMICROBIAL AGENTS, ELSEVIER, AMSTERDAM, NL, vol. 25, no. 1, 1 January 2005 (2005-01-01), pages 11-25, discloses colistin (polymyxin E) as an agent againts multi-resistant Gram negative bacteria.

### SUMMARY OF THE PRESENT INVENTION

The invention is set out in the appended set of claims.

The description may encompass subject-matter extending beyond the scope of the claims. However the scope of the invention and of protection is solely defined by theappended claims. In particular, the scope of protection does not include any method of therapeutic treatment of the human or animal body, even if such subject-matter is disclosed or implied herein. The references to methods of treatment in the subsequent paragraphs of this description are to be interpreted as references to the compounds of the present invention, pharmaceutical composition and medicaments disclosed herein for use in those methods.

To fill the gap in the application of compounds with benzoyl aniline as the parent nucleus structure in antibacterial fields, and to propose a fully synthesized benzoyl aniline compound with independent intellectual property rights for antibacterial applications, the compound BAB159 with the best effect was chosen. The compound provided by the present invention has a broad antibacterial spectrum, good antibacterial activity, suitable in vivo pharmacokinetics, and significant in vivo therapeutic effects.

Meanwhile, the present invention also discovered that BAB159 and polymyxin E have a synergistic effect. The combination of the two in an appropriate proportion has superior antibacterial activity, especially the activity of multidrug-resistant bacteria. In the current environment where various bacteria are gradually developing resistance to common antibiotics, it has extremely important practical significance and clinical research needs.

The present disclosure provides a compound, having the following formula (I): 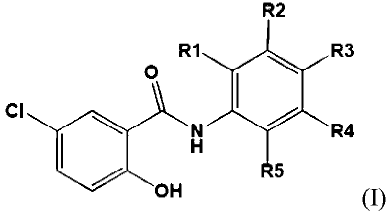 wherein, R₁, R₂, R₃, R₄, and R₅ are at least one selected from H, C₁₋₄ alkyl,C₁₋₄ alkoxy, halogen, hydroxyl, and nitro; wherein H atoms on the C₁₋₄ alkyl and/or the C₁₋₄ alkoxy are optionally replaced by halogen atom or halogen atoms, provided that at least two of R₁, R₂, R₃, R₄, and R₅ are halogens, but not more than four.

Preferably, at least one of R₁, R₂, R₃, R₄, and R₅ is CF₃, but not more than two.

Not being a part of the present invention, the compound with broad-spectrum antibacterial activity is the following specific compound:

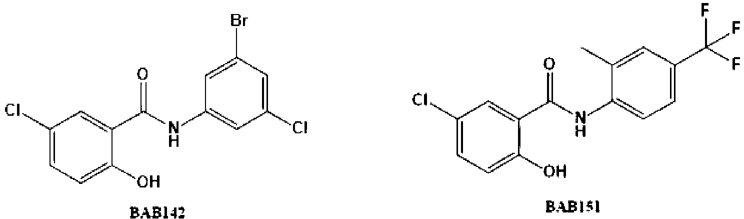

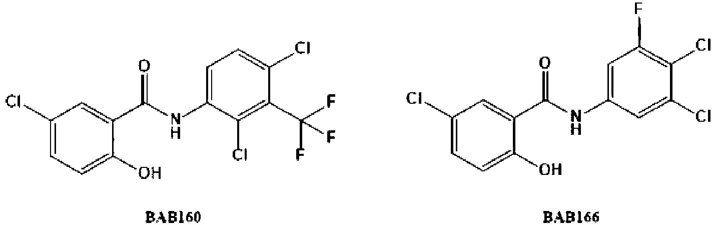

After extensive screening, the inventor selected the specific compounds mentioned above as representative benzoyl aniline derivatives with broad-spectrum antibacterial activity in the present invention. Preferably, compound BAB159 exhibits the strongest antibacterial activity and has a broad-spectrum antibacterial activity, superior to most known commercial conventional antibiotics.

The present invention provides a compound having a structure as shown in the following compound BAB 159

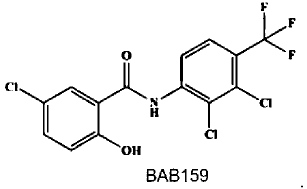

The present disclosure also provides an antibacterial composition, comprising the following active ingredients: the compound shown in above formula (I), and polymyxin.

Furthermore, in the antibacterial composition, concentration ratio of the compound shown in formula (I) to polymyxin is 1-3:1-3. The concentration ratio is the mass concentration ratio. The mass concentration of the compound shown in formula (I) varies for different bacteria, generally ranging from 0.01 to 16 µg/mL, preferably 0.1 to 1 µg/mL, such as 0.5 µg/mL.

Furthermore, the polymyxin is at least one selected from polymyxin A, polymyxin B, polymyxin C, polymyxin D, and polymyxin E; preferably polymyxin E.

In the present invention, the active ingredients of the antibacterial composition are BAB 159 and polymyxin E, wherein a concentration ratio of BAB159 to polymyxin E is 1-3:1-3.

The inventor unexpectedly found that the compound BAB159 of the present invention, in combination with polymyxin, particularly polymyxin E, exhibits significant synergistic antibacterial activity (FIC<0.078) against multidrug-resistant *E. coli* B2, particularly against multidrug-resistant strains. However, such a strong synergistic antibacterial activity was not observed in BAB159 and other antibacterial compounds such as ciprofloxacin, kanamycin, and polymyxin B.

The present invention also provides the compound or the composition for use in anti-bacterial and/or anti-fungal treatment.

Furthermore, the bacteria comprising at least one selected from Staphylococcus, Clostridium perfringens, Enterococcus, Bacillus, Streptococcus, Haemophilus, and Mycobacterium smegmatis; the fungi comprising at least one selected from Candida, Aspergillus niger, Aspergillus flavus, and Trichophyton.

The excellent effects of the present invention are:
1. The present invention provides a novel antibacterial drug source compound with a broad antibacterial spectrum, good antibacterial activity, suitable in vivo pharmacokinetics, and significant in vivo therapeutic effects for clinical use. The compound BAB159 has a minimum inhibitory concentration (MIC₅₀) of 0.01 µg/mL, 0.25 µg/mL, 0.25 µg/mL, 0.01 µg/mL, 0.125 µg/mL, and 16 µg/mL for Staphylococcus (including MRSA), Clostridium perfringens, Enterococcus, Bacillus, Streptococcus, and Haemophilus, respectively. The MIC values for Candida krusei ATCC 6258, Aspergillus niger, Aspergillus flavus, and Trichophyton were 1 µg/mL, 0.25 µg/mL, 1 µg/mL, and 0.125 µg/mL, respectively. The antibacterial activity of BAB159 provided by the present invention against common clinical pathogens is comparable or superior to the listed drugs linezolid, ceftiofur, penicillin, vancomycin, lincomycin, doxycycline, trimethoprim, tiamulin, quinolone, and amphotericin B. The compounds provided by the present invention enrich the molecular library of the antibacterial drug source, alleviate the severe situation of drug resistance, and provide a new treatment option for infections caused by clinically resistant pathogens. In the current reality where more and more bacteria are developing resistance to antibiotics, it has extremely important scientific research value and clinical practical significance.
2. It was also unexpected to find that the combination of BAB159 compound and polymyxin E exhibits significantly enhanced synergistic antibacterial activity, particularly against multidrug-resistant strains. Therefore, the antibacterial composition with BAB159 compound and polymyxin E as active ingredients may play a significant role.

### BRIEF DESCRIPTION OF THE DRAWINGS

**Figure 1** shows the ¹H NMR of compound BAB159 in DMSO solvent;
**Figure 2** shows the ¹³C NMR of compound BAB159;
**Figure 3** shows the high-resolution mass spectrum of compound BAB159 in positive ion mode;
**Figure 4** shows the high-resolution mass spectrum of compound BAB159 in negative ion mode;
**Figure 5** shows the synergistic antibacterial activity of compound BAB159 in combination with polymyxin E on multidrug-resistant bacteria *E. coli* B2;
**Figure 6** shows the growth curves of Staphylococcus aureus ATCC 29213, MRSA T144, and vancomycin resistant Enterococcus faecium CAU369 under the action of BAB159;
**Figure 7** shows the time-Kill curve of BAB159 against Staphylococcus aureus ATCC 25923;
**Figure 8** shows the protective efficacy of BAB159 against infection by the Galleria mellonella;
**Figure 9** shows the survival rate of infected mice under the action of BAB159.

### DETAILED DESCRIPTION OF THE INVENTION

In order to clarify the purpose, technical solution, and advantages of the present invention, a detailed description of the technical solution of the present invention will be provided below. The following embodiments facilitate a better understanding of the present invention, but do not limit the present invention. The experimental methods in the following embodiments, unless otherwise specified, are all conventional methods.

The standard strains used in the present invention, such as Staphylococcus aureus ATCC 29213 and Candida krusei ATCC 6258, were purchased from the National Standard Material Network. Clinical strains, such as A57-1, B58-2, A3-2, B4 and B5, were isolated from animal tissues, excreta, and feeding environments. The above strains are stored in the National Key Laboratory of Veterinary Public Health and Safety as required.

### Preparation Example

Taking compound BAB159 as an example, the synthesis strategy for preparing compound (I) of the present invention is illustrated. When preparing other compounds, it is only necessary to replace the raw materials with different substituents to obtain the other compounds through the same synthesis strategy for preparing BAB 159.

The synthesis route of compound BAB159 is as follows:

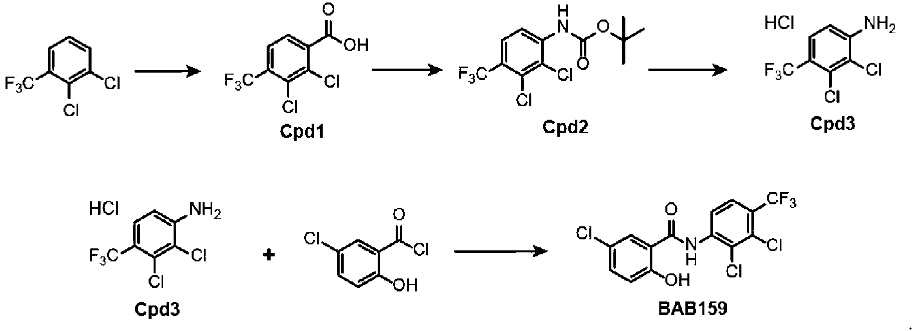

Specifically, it includes the following steps:
(S1) Add tetrahydrofuran to a there-necked flask and stir to replace nitrogen. Cool down to -75 °C. Add 29.8 g butyl lithium (1 eq) and 47.1 g diisopropylamine (1 eq) dropwise at controlled temperature and stir for 10 minutes. Then add 100 g of 1,2-dichloro-3-(trifluoromethyl)benzene (1 eq) dropwise and incubate for two hours after the addition. The reaction solution was poured into dry ice, naturally heated to room temperature and stirred overnight. The reaction solution was concentrated to remove tetrahydrofuran, and 100mL of water and 200mL of ethyl acetate were added. The acid was adjusted to pH 3, and the liquid was separated. The organic phase was concentrated to obtain the crude product, which was then slurried with n-hexane and filtered to obtain 72 g of intermediate product Cpd1 with a yield of 60%.
(S2) Add 72 g (1 eq) of Cpd1 and 720 mL of dioxane to a single-necked flask to dissolve evenly. Then, add 350 mL of tert-butanol, 112 g (4 eq) of triethylamine, and 115 g of diphenylphosphoryl azide (1.5 eq) in sequence. After nitrogen replacement three times, react overnight. Pour the reaction solution into ice, concentrate with ethyl acetate for extraction, and stir the organic phase (petroleum ether: ethyl acetate=20:1) to obtain 72 g of intermediate product Cpd2 with a yield of 78.4%.
(S3) Add 72g of Cpd2 to a single-necked flask, dissolve in 720 mL of ethyl acetate, and then add 720 mL of saturated hydrochloric acid ethyl acetate and stir overnight. The white solid that gradually precipitates is the product. The solid obtained by filtration is 57 g of intermediate product Cpd3 with a yield of 98%.
(S4) Dissolve 100 g of salicylic acid in 1 L of dichloromethane, add 8 mL of N, N-dimethylformamide, and add 1.2 eq of oxalyl chloride under ice bath conditions. React at room temperature for 2 hours until the reaction becomes clarified. After the central control is completed, spin-dry to obtain the acyl chloride compound, which is ready for use.
(S5) Add 50 g (1 eq) of Cpd3 and acetonitrile to a single-necked flask and stir evenly, add potassium iodide (62 g (2 eq)), and slowly add 43 g (dissolved in acetonitrile) of the acyl chloride compound (1.2 eq) prepared in step (S4), and react at 80 °C for 16 hours. Let it stand overnight until a solid precipitate is formed. After filtration, dissolve the filter cake in ethyl acetate and wash it twice with water. Dry the organic phase and spin-dry the resulting product, which was then slurried with dichloromethane and filter the solid to obtain product BAB159 with a yield of 38.4%.

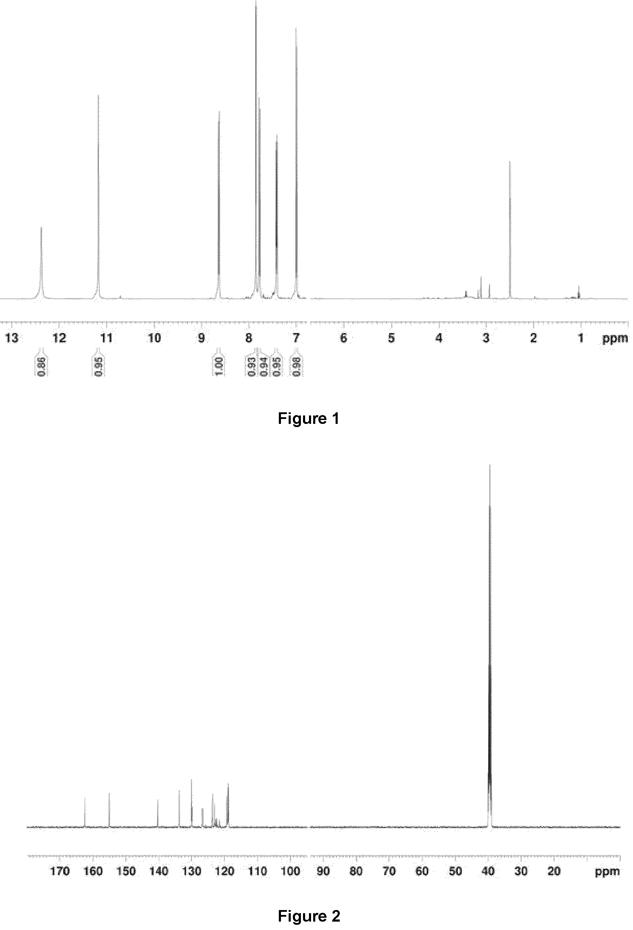

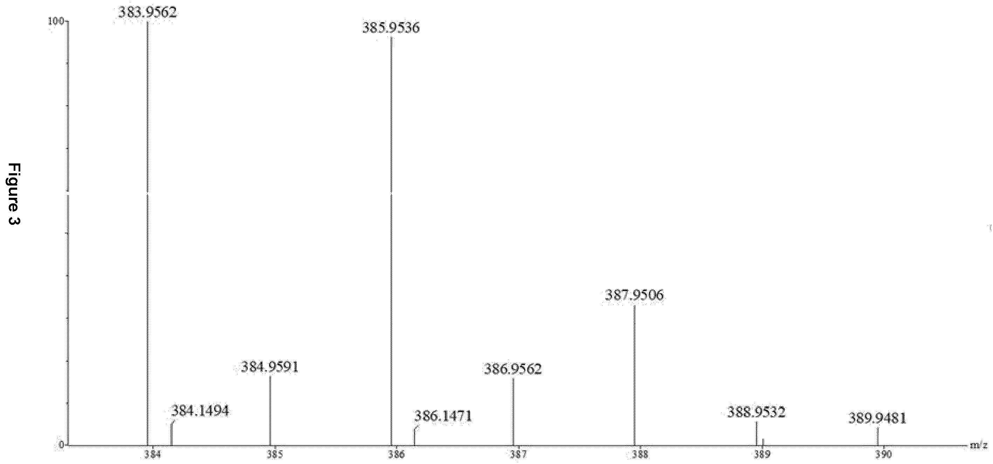

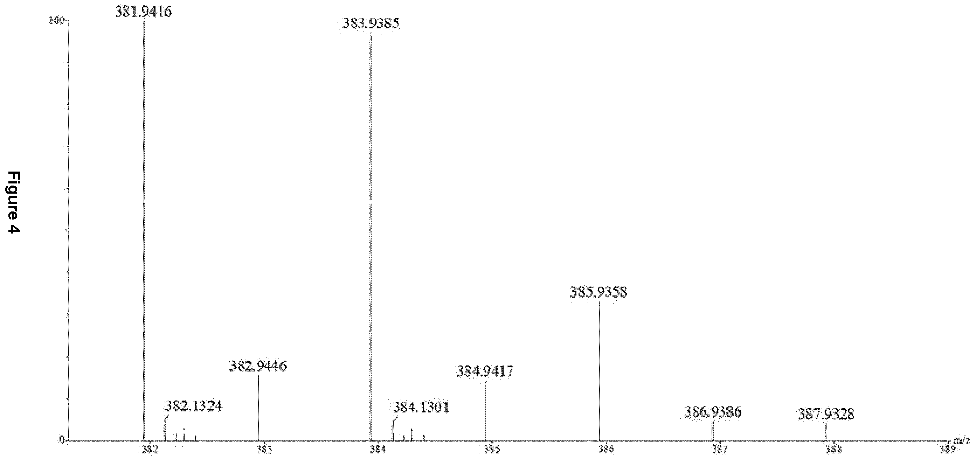

Figure 1 shows the ¹H NMR of compound BAB159 in DMSO solvent. Figure 2 shows the ¹³C NMR of compound BAB159. Figure 3 shows the high-resolution mass spectrum of compound BAB159 in positive ion mode. Figure 4 shows the high-resolution mass spectrum of compound BAB159 in negative ion mode. From Figure 1 to Figure 4, it can be determined that the structure of compound BAB159 obtained in S5 is as follows:

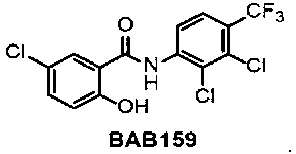

### Embodiment 1 Antibacterial activity of BAB159 against common clinical pathogenic bacteria and fungi

Accurately weigh the compound BAB159 prepared in the preparation example, dissolve it appropriately in dimethyl sulfoxide (DMSO), add 100 µL of MHB broth culture medium to a 96 well U-shaped plate, take 100 µL of a certain concentration of the compound and add it to the first column of the 96-well U-shaped plate, and dilute it to the 10th column by multiple ratio. Select single colonies of the test strain and culture them in BHI broth on a shaker at 37 °C until the bacterial logarithmic growth stage. Adjust the bacterial turbidity to 0.5 McF using a Maxwell turbidimeter and dilute 100-fold (approximately 10⁶ CFU/mL) with MHB broth culture medium. Take 100 µL of the above bacterial solution and add it to a 96 well U-shaped plate. The 11th and 12th columns only contain MHB broth culture medium and the test bacterial solution, respectively, as negative and positive controls. Place the 96-well U-shaped plate in a 37 °C constant temperature incubator for 16-18 hours, read the experimental results, and determine the minimum inhibitory concentration (MIC) of the compound as the visible lowest drug concentration that inhibits bacterial growth. The results are shown in Tables 1 and 2 below.

The number of more types of bacteria and strains were determined by compound BAB159, the results are shown in Table 1. Compound BAB159 has good antibacterial activity against pathogenic bacteria including Staphylococcus (50 strains), Enterococcus (27 strains), Bacillus (21 strains), Clostridium perfringens (35 strains), Streptococcus (30 strains), and Mycobacterium smegmatis (1 strain), with MIC values of 0.01 µg/mL, 0.25 µg/mL, 0.01 µg/mL, 0.25 µg/mL, 0.125 µg/mL, and 2 µg/mL, respectively. BAB159 has better or equivalent antibacterial activity than vancomycin and linezolid.

**Table 1 Antibacterial efficacy of BAB159 against common clinical pathogenic bacteria (MICs, unit: µg/mL)**

| Project | Staphylococcus (n=50) | Enterococcus (n=27) | Bacillus (n=21) | Clostridium perfringens (n=35) | Streptococcus (n=30) | Mycobacterium smegmatis (n=1) |
|---|---|---|---|---|---|---|
| BAB159 | 0.01 | 0.25 | 0.01 | 0.25 | 0.125 | 2 |
| Positive control | 1 | 1 | 0.5 | 0.5 | 0.25 | 16 |

| | | | | | | |
|---|---|---|---|---|---|---|
| Note: Except the positive control for Enterococcus, which is linezolid, the positive control for all other strains is vancomycin. | | | | | | |

Meanwhile, compound BAB159 also exhibits strong antibacterial activity against common clinical pathogenic fungi such as Candida albicans, Aspergillus flavus, Aspergillus niger, and Trichophyton, with a MIC of 0.5-1 µg/mL. In addition, BAB159 also has certain antibacterial activity against gram-negative bacteria such as Haemophilus, with a MIC of 8-32 µg/mL. Based on the above research results, BAB159 provided by the present invention has better or equivalent antibacterial activity against different bacterial genera such as Gram-positive bacteria, some Gram-negative bacteria, and fungi than listed drugs. Its further application will undoubtedly be beneficial for the prevention and control of related diseases in clinical practice.

**Table 2 Antibacterial efficacy of BAB 159 against common clinical pathogenic fungi (MICs, unit: µg/mL)**

| Strains | MICs (µg/mL) | |
|---|---|---|
| | BAB159 | Amphotericin B |
| Candida albicans ATCC 6258 | 1 | 0.5 |
| Aspergillus flavus ATCC 11492 | 1 | 0.25 |
| Aspergillus niger ATCC 96918 | 0.5 | 0.125 |
| Trichophyton ATCC MTA-4439 | 0.125 | 0.25 |

### Embodiment 2

The antibacterial activity of BAB159, BAB142, BAB151, BAB160, and BAB166 against Clostridium perfringens was tested (n=26). The results are shown in Table 3 below.

**Table 3 Antibacterial activity of compounds with different structures against Clostridium perfringens (MIC₅₀, unit: µg/mL)**

| BAB159 (Example) | BAB142 (Comparative example) | BAB151 (Comparative example) | BAB160 (Comparative example) | BAB166 (Comparative example) |
|---|---|---|---|---|
| 0.25 | 16 | 8 | 8 | 32 |

It can be seen that among the compounds of formula (I), BAB159 has the best antibacterial activity with a MIC₅₀ of 0.25 µg/mL. Other similar compounds, such as BAB142, BAB151, BAB160, and BAB166, also have certain antibacterial activity and can meet the antibacterial applications of different drug-resistant bacteria/fungi.

### Embodiment 3 Comparison of antibacterial activity of BAB159 and some listed antibiotics against Clostridium perfringens

Take out the A-type standard strain A57-1 (CVCC 2030), B-type standard strain B58-2 (CVCC 60081), C-type standard strains C59-1 and C59-2 (CVCC 60101, CVCC 60102), D-type standard strain 60-2D (CVCC 60201), and E-type strain E4 of Clostridium perfringens purchased from the National Standard Material Network in an -80 °C freezer, thaw at room temperature, and apply them to BHA blood plates for streaking and recovery. Take out 26 clinical isolates from farms, such as A57-1, B58-2, A3-2, B4 and B5, in a -80 °C freezer, thaw at room temperature, and apply them to BHA blood plates for streaking and recovery. Culture at 37 °C in anaerobic environment for 20-24 hours, pick 3-5 single colonies with an inoculation ring, inoculate them in FTG culture medium, and let them stand at 37 °C in anaerobic environment for 12 hours. After the culture was completed, a drug sensitivity test was carried out. Adjust bacterial turbidity to 0.5 McF using a Maxwell turbidimeter and dilute 100-fold with FTG culture medium (at this time, the bacterial concentration is about 1 × 10⁶ CFU/mL) for later use. Dilute the test drug to 10 concentration gradients using FTG culture medium, take 100 µL and add it to a 96 well U-shaped plate. Then add 100 µL of spare bacterial solution to each well, and the 11th and 12th columns are used as negative controls containing FTG culture medium only and positive controls containing bacterial solution only, respectively. Place the 96-well U-shaped plate that has completed bacterial solution inoculation in anaerobic culture at 37 °C for 12-16 hours and read the drug sensitivity results. In this experiment, Bacteroides fragilis (ATCC 25285) was used as the quality control bacterium. As shown in Table 4, most of the tested antibiotics have high MICs against Clostridium perfringens. The MIC₅₀ of lincomycin against Clostridium perfringens is 8 µg/mL, and the MIC₉₀ is greater than 128 µg/mL. The MIC₅₀ and MIC₉₀ of doxycycline are 8 µg/mL and 16 µg/mL, respectively. The MIC₅₀ and MIC₉₀ of tilmicosin are 128 µg/mL and greater than 128 µg/mL, respectively. The MIC₅₀ and MIC₉₀ of tiamulin is 0.5 µg/mL and 8 µg/mL, respectively. The compound BAB159 provided by the present invention has excellent antibacterial effect on Clostridium perfringens, with MIC₅₀ and MIC₉₀ of 0.25 µg/mL and 0.5 µg/mL, respectively. Compared with commonly used antibiotics in clinical practice, BAB159 has the highest MIC value of only 0.5 µg/mL against 26 tested strains of Clostridium perfringens, which was comparable to the antibacterial activity of ceftiofur, penicillin, and vancomycin. This indicates that the compound has comparable or even better efficacy compared to listed commonly used antibiotics and has good application prospects in the treatment of drug-resistant anaerobic Clostridium infections.

**Table 4 Activity of BAB 159 and some listed antibiotics against Clostridium perfringens**

| Type | Strain number | **MIC value against Clostridium perfringens (µg/mL)** | | | | | | | | |
|---|---|---|---|---|---|---|---|---|---|---|
| | | Example | Comparative Examples | | | | | | | |
| | | BAB159 | CEF | PEN | VAN | LIN | DOX | TMS | TIA | QCT |
| A-type | A57-1 | 0.25 | ≤0.25 | 0.25 | ≤0.25 | 2 | ≤0.25 | 4 | 4 | ≤0.25 |
| | A3-1 | 0.5 | ≤0.25 | 0.25 | ≤0.25 | 1 | 8 | 4 | 0.5 | 0.5 |
| | A3-2 | 0.25 | ≤0.25 | 0.5 | ≤0.25 | 0.5 | 8 | 2 | 0.5 | ≤0.25 |
| B-type | B4 | 0.5 | ≤0.25 | 0.5 | ≤0.25 | >128 | 2 | >128 | 4 | ≤0.25 |
| | B5 | 0.25 | ≤0.25 | 0.5 | ≤0.25 | >128 | 8 | >128 | 0.5 | ≤0.25 |
| C-type | C3 | 0.5 | 1 | 2 | 0.5 | >128 | 16 | >128 | 8 | 1 |
| | C8 | 0.25 | ≤0.25 | 0.25 | ≤0.25 | 128 | ≤0.25 | >128 | 0.5 | ≤0.25 |
| | C59-1 | 0.25 | ≤0.25 | 0.125 | ≤0.25 | 0.5 | ≤0.25 | 2 | ≤0.25 | ≤0.25 |
| D-type | D14B | 0.25 | ≤0.25 | 0.5 | ≤0.25 | 64 | 1 | 128 | 8 | ≤0.25 |
| F-type | F5-2 | 0.25 | ≤0.25 | 0.125 | ≤0.25 | 128 | 4 | 128 | 0.5 | 0.5 |
| | F6-1 | 0.25 | ≤0.25 | 0.25 | 0.5 | 32 | 32 | 128 | 0.5 | 4 |
| | F6-2 | 0.5 | ≤0.25 | 0.125 | ≤0.25 | 32 | 16 | 128 | 0.5 | 0.5 |
| | F6-2-1 | 0.5 | ≤0.25 | 0.25 | ≤0.25 | 128 | 16 | >128 | 0.5 | 1 |
| | F6-2-2 | 0.25 | ≤0.25 | 0.25 | ≤0.25 | 128 | 16 | 128 | 0.5 | ≤0.25 |
| | F7-1 | 0.5 | ≤0.25 | 0.5 | 0.5 | >128 | 8 | 64 | 4 | ≤0.25 |
| | F8-1 | 0.5 | ≤0.25 | 0.25 | 0.5 | >128 | 16 | 128 | 4 | 0.5 |
| H-type | H12-1 | 0.25 | ≤0.25 | 0.25 | ≤0.25 | 0.5 | 4 | 4 | 0.5 | ≤0.25 |
| | H12-2 | 0.25 | ≤0.25 | 0.125 | ≤0.25 | ≤0.25 | 4 | 4 | ≤0.25 | ≤0.25 |
| | H13-1 | 0.5 | ≤0.25 | 0.0625 | ≤0.25 | 4 | 8 | 4 | 4 | ≤0.25 |
| | H14-1 | 0.5 | ≤0.25 | 0.25 | 0.5 | 8 | 8 | 128 | 8 | ≤0.25 |
| | H14-2 | 0.5 | ≤0.25 | 1 | ≤0.25 | 8 | 8 | 128 | 8 | 0.5 |
| | H14-4 | 0.5 | ≤0.25 | 0.125 | ≤0.25 | 4 | 4 | 128 | 4 | ≤0.25 |
| | H15-1 | 0.25 | ≤0.25 | 0.25 | 0.5 | ≤0.25 | 16 | 4 | ≤0.25 | ≤0.25 |
| | H15-2 | 0.5 | ≤0.25 | 0.25 | ≤0.25 | 4 | 16 | 128 | 4 | ≤0.25 |
| | H15-3 | 0.25 | ≤0.25 | 0.25 | ≤0.25 | 4 | 8 | 4 | 4 | 0.5 |
| | H16 | 0.25 | ≤0.25 | 0.125 | 0.5 | 4 | 8 | 128 | 4 | ≤0.25 |

| | | | | | | | | | | |
|---|---|---|---|---|---|---|---|---|---|---|
| Note: CEF: Ceftiofur, PEN: Penicillin, VAN: Vancomycin, LIN: Lincomycin, DOX: Doxycycline, TMS: Tilmicosin, TIA: Tiamulin, QCT: Quincetone. | | | | | | | | | | |

### Embodiment 4 Synergistic Antibacterial Efficacy of BAB159 and Polymyxin E

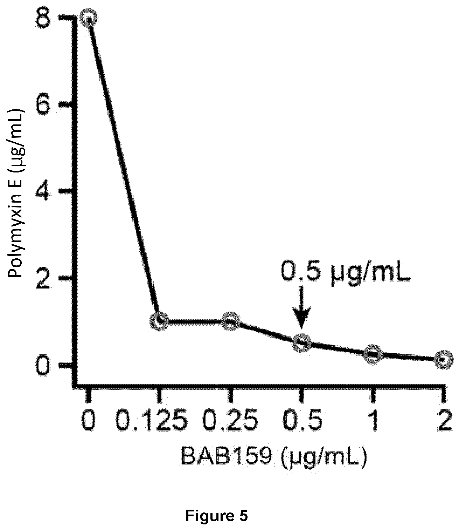

Using the chessboard broth dilution method for the combined treatment of BAB159 and polymyxin E against multidrug-resistant *E. coli* B2_{(NDM-5+MCR-1)} was tested for synergistic efficacy (FIC). *E. coli* B2 single colony was picked and cultured in BHI broth on a shaker at 37 °C until logarithmic growth stage. Adjust the bacterial turbidity to 0.5 McF using a Maxwell turbidimeter and dilute 100-fold (~1.0 × 10⁶ CFU/mL) with MHB culture medium for future use. Add 100 µL of polymyxin E with a concentration of 32 µg/mL to each well in the first column. Use a pipette to take 100 µL of the solution in sequence and dilute it 2-fold from the 1st column to the 7th column. Discard the excess liquid. Add 100 µL of BAB159 with a concentration of 2 µg/mL to each well in the 8th row. Use a pipette to take 100 µL of the solution and dilute it 2-fold from the 8th row to the 2nd row. Discard excess liquid. Subsequently, add 100 µL of diluted tested bacterial solution to each well from 1st column to 8th column. 11th column and 12th column represent negative and positive controls containing only MHB culture medium and only tested bacterial solution, respectively. The result is shown in Figure 5.

Result interpretation: after incubation at 37 °C for 16-18 hours, read the FIC value, where FIC=MIC (polymyxin E when combined)/MIC (polymyxin E when used alone) + MIC (BAB159 when combined)/MIC (BAB159 when used alone). Where FIC>2 indicates antagonistic effect, 1<FIC≤2 indicates irrelevant effect, 0.5<FIC≤1 indicates additive effect, and FIC≤0.5 indicates synergistic effect.

The results shows that when polymyxin E and BAB159 are used alone, MICs of polymyxin E and BAB159 against multidrug-resistant *E. coli* B2 are 8 µg/mL and >32 µg/mL, respectively. When used in combination, polymyxin E and BAB159 have a significant synergistic effect at a concentration ratio of 1-3:1-3. The best synergistic effect is achieved when polymyxin E and BAB159 are used in a 1:1 concentration ratio. At this time, MICs of polymyxin E and BAB159 against *E. coli* B2 are both 0.5 µg/mL. At this ratio, BAB159 increases the MIC of polymyxin E by 16-fold and the FIC index is less than 0.078. BAB159 also shows a synergistic effect when combined with polymyxin B (FIC=0.5), but the effect is not as strong as polymyxin E, and no synergistic effect was observed when combined with ciprofloxacin and kanamycin (FIC=2).

This demonstrates that BAB159 provided by the present invention can be used in combination with polymyxin E to treat multidrug-resistant *E. coli* B2 exerts a synergistic antibacterial effect. The above results indicate that BAB159 not only has superior antibacterial activity on its own, but also can be combined with polymyxin to enhance its activity against pathogenic bacteria, especially multidrug-resistant bacteria. BAB159 provided by the present invention has good clinical application prospects.

**Table 5 Synergistic effect of BAB 159 against polymyxin E (MICs unit: µg/mL)**

| Drug A | MICs (drug A when used alone) | MICs (drug A when used in combination) | MICs (BAB159 when used alone) | MICs (BAB159 when used in combination) | Synergistic index (FIC) |
|---|---|---|---|---|---|
| Polymyxin E | 8 µg/mL | 0.5 µg/mL | >32 µg/mL | 0.5 µg/mL | <0.078 |
| Polymyxin B | 8 µg/mL | 2 µg/mL | >32 µg/mL | 8 µg/mL | 0.5 |
| Polymyxin C | 16 µg/mL | 4 µg/mL | >32 µg/mL | 16 µg/mL | 0.75 |
| Ciprofloxacin | 32 µg/mL | 32 µg/mL | >32 µg/mL | >32 µg/mL | 2.0 |
| Kanamycin | 128 µg/mL | 128 µg/mL | >32 µg/mL | >32 µg/mL | 2.0 |

### Embodiment 5 Growth curves of common pathogenic bacteria under the action of BAB159

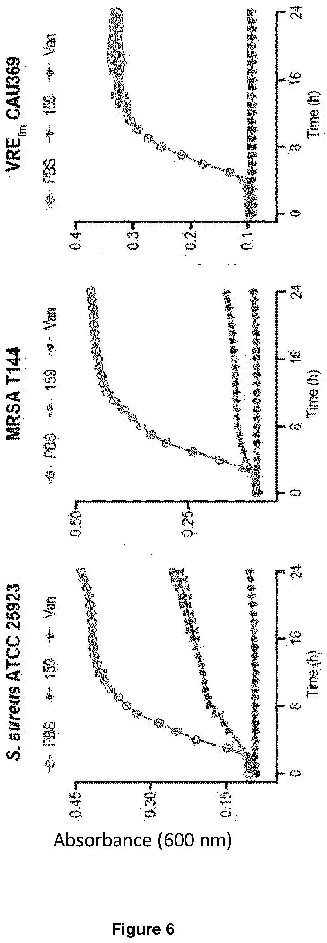

Select strains of Staphylococcus aureus ATCC 25923, MRSA T144, and vancomycin resistant Enterococcus faecium CAU369 for monoclonal transfer to BHI broth culture medium, and culture on a shaker at 37 °C and 200 rpm until logarithmic growth stage. Adjust the bacterial turbidity to 0.5 McF using a Maxwell turbidimeter and dilute 100-fold (~1.0 × 10⁶ CFU/mL) with MH broth culture medium for future use. Dilute BAB159 to the desired concentration using MH broth culture medium, take 100 µL of BAB159 and add it to a 96-well flat plate. Add 100 µL of diluted bacterial solution to each well. Set up negative and positive controls containing only MH broth culture medium and the test bacterial solution. Set the temperature of the enzyme-linked analyzer system to 37 °C and measure the absorbance at OD 600 nm once per hour for a total of 24 hours. Three biological replicates were set for each treatment, as shown in Figure 6. In Figure 6, PBS represents the buffer group, 159 represents the compound BAB159 group, and van represents the vancomycin group. By observing the dynamic curve of bacterial growth, it can be more intuitively observed that compound BAB159 can significantly inhibit the growth of Staphylococcus aureus ATCC 25923, MRSA, and vancomycin resistant Enterococcus faecium CAU369 at its MIC concentration. The growth inhibition effect on vancomycin resistant Enterococcus faecium CAU369 is comparable to that of vancomycin.

### Embodiment 6 Time-Kill curve of BAB159

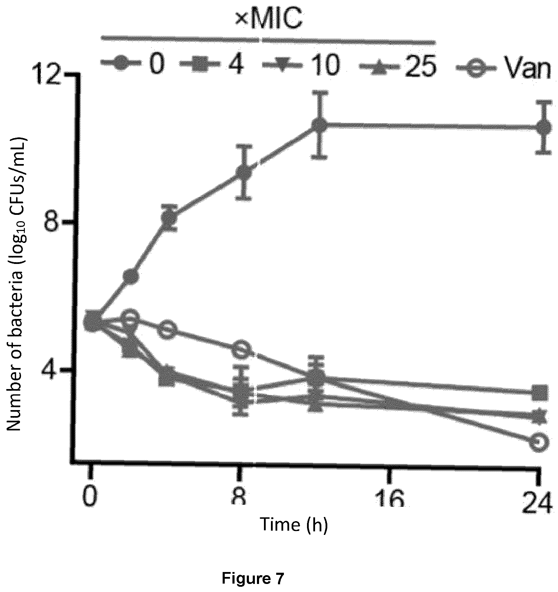

Select monoclonal Staphylococcus aureus ATCC 25923 and culture it in 1 mL BHI broth culture medium at 37 °C and 200 rpm until logarithmic growth stage. Then, adjust the concentration of Staphylococcus aureus ATCC 25923 to 0.5 McF, and dilute it 100-fold to 5 mL of prepared MH broth to obtain a bacterial solution with a bacterial concentration of about 10⁶ CFU/mL. Add different concentrations of 159 (4×MIC, 10×MIC, and 25×MIC) to the bacterial broth, and add 10×MIC vancomycin (Van) and PBS buffer (0×MIC) to the bacterial broth as positive and negative control groups, respectively. Set 3 replicates for each treatment and incubate in a shaker at 37 °C and 200 rpm. Take out 100 µL of culture medium after the specific time point of the addition of antibacterial compounds, dilute it in PBS at a multiple ratio, coat and count, and invert at 37 °C for 24 hours to calculate the number of colonies. As shown in Figure 7, with the increase of BAB159 concentration, its antibacterial efficacy did not significantly increase, but over time, the antibacterial effect became more significant at each concentration. Prove that BAB159 is a time-dependent fungicide. For time-dependent antibiotics, the pharmacokinetic/pharmacodynamic (PK/PD) parameter to be selected for dosage formulation is the ratio of the area under the drug time curve (AUC) to the MIC. Therefore, the AUC to MIC ratio was chosen for the subsequent formulation of BAB159 dosage.

### Embodiment 7 Pharmacokinetic characteristics of BAB159

In order to investigate the metabolic residues of BAB159 in model animals, rabbits were used as animal models to quantitatively analyze the levels of BAB159 in rabbit plasma. Male Chinese white rabbits weighing around 2 kg were adaptively fed for one week. Then, 2 mL of BAB159 suspension was injected into the left hind thigh muscle of the rabbits at a dose of 20 mg/kg b.w. Blood samples were collected from the ear vein at 8 time points, including 0.5, 1, 2, 4, 6, 8, 12, and 24 hours after administration. After pre-treatment of the plasma samples, UPLC-MS/MS detection was performed.

The results shows that after intramuscular injection, the Cₘₐₓ, Tₘₐₓ, and AUC in the plasma of rabbits are 207 µg/L, 6 h, and 1708 µg/L·h, respectively (Table 6). According to the antibacterial spectrum of BAB159, the MIC₅₀ of the compound against Staphylococcus is 15.6 µg/L. Pharmacokinetic parameters show that the area under the drug time curve AUC after intramuscular injection is 1708 µg/L·h, obtain AUIC=109. Research has shown that when the AUIC of time-dependent antibiotics is greater than 100, the compound is considered to have superior therapeutic effects (Pharmaceutics, 2021, 13(5): 602). The above results indicate that the compound BAB159 of the present invention has great potential for application in the treatment of clinically relevant pathogenic infections.

**Table 6 Pharmacokinetic parameters of BAB159 in rabbits**

| Parameter | Unit | Numerical value |
|---|---|---|
| Dose | mg/kg b.w | 20 |
| Tₘₐₓ | h | 6 |
| Cₘₐₓ | µg/L | 148.2±13.05 |
| Vd | L | 0.28±0.03 |
| AUC₀₋ₗₐₛₜ | µg/L·h | 1644.8±70.5 |
| T_{1/2} | h | 16.1±3.6 |
| CL | mL/min | 0.01±0.003 |

| | | |
|---|---|---|
| Note: Tₘₐₓ: time to peak; Cₘₐₓ: maximum blood drug concentration; Vd: apparent distribution volume; AUC: area under the drug time curve; T_{1/2}: half-life; CL: clearance constant. | | |

### Embodiment 8 Efficacy of compound BAB159 in an in vivo animal infection model

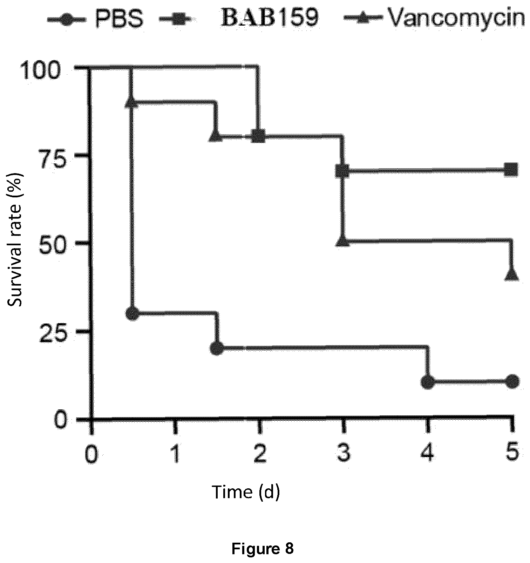

In vivo infection model of the Galleria mellonella: randomly divide approximately 300 mg of Galleria mellonella larvae into 8 individuals per group. Each Galleria mellonella was injected with 10 µL of MRSA T144 bacterial suspension (final concentration of 5.5 × 10⁶ CFU/individual) from its left proleg. After 1 hour of bacterial infection, 10 µL of BAB159 solution was injected into the right proleg of the Galleria mellonella at a dose of 2 mg/kg b.w. The positive control was vancomycin with a concentration of 2 mg/kg b.w. Record the survival rate of each group of the Galleria mellonella daily for 5 days. Figure 8 shows the protective efficacy of compound BAB159 against infection by the Galleria mellonella. In the infection model of the Galleria mellonella, the protective rate of BAB159 against the Galleria mellonella within 5 days of infection was 70%, which was better than the 40% of vancomycin.

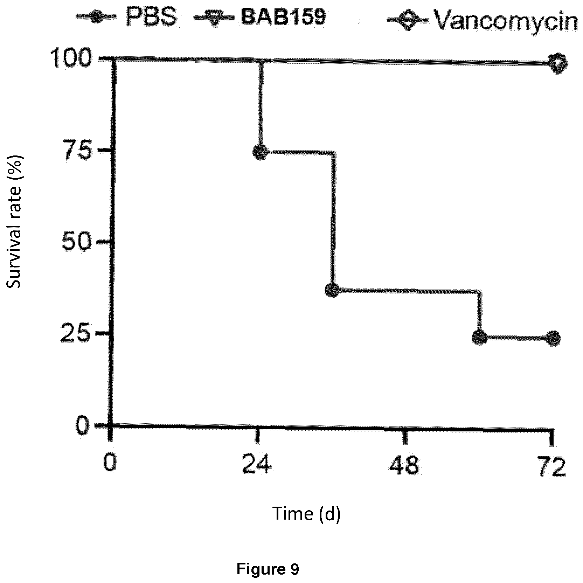

Mouse bacteremia model: C57BL/6J female mice weighing between 18-20 g were used. After one week of adaptive feeding, the mice were randomly divided into 5 groups, with 7 mice in each group. Each mouse was intraperitoneally injected with 200 µL of MRSA T144 bacterial suspension (final concentration of 1.5×10⁸ CFU/individual). After 1 hour of bacterial infection, mice were administered corresponding compounds and positive control vancomycin at a dose of 10 mg/kg b.w. Record the survival rate of mice within 72 hours. Figure 9 shows the survival rate of mice infected with BAB159. In the mouse infection model, the protective rate of BAB159 on mice within 3 days was 100%, which was comparable to the efficacy of vancomycin and significantly stronger than the PBS group.

The above results indicate that BAB159 provided by the present invention has excellent antibacterial activity against common clinical pathogenic bacteria and fungi, and its effect is superior to some listed antibiotics. At the same time, the pharmacokinetic parameters of BAB159 are suitable and have shown good therapeutic effects on both the Galleria mellonella and rabbit infection models, indicating that BAB159 can be used for the treatment of related pathogenic bacterial infections and has good clinical application prospects.

## Claims

1. A compound, having a structure as shown in the following compound BAB159:

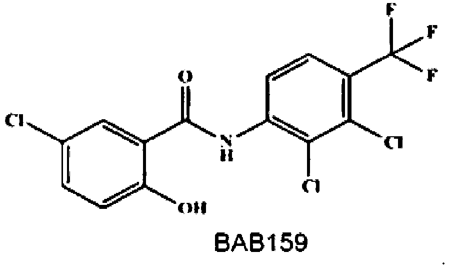

2. A composition, comprising the compound BAB159 of claim 1 and polymyxin E as active ingredients;
wherein a concentration ratio of BAB159 to polymyxin E is 1-3:1-3.

3. The compound according to claim 1 or the composition according to claim 2 for use in anti-bacterial and/or anti-fungal treatment.

4. The compound or the composition for use according to claim 3, wherein the bacteria include at least one selected from Staphylococcus, Clostridium perfringens, Enterococcus, Bacillus, Streptococcus, Haemophilus, and Mycobacterium smegmatis; and the fungi include at least one selected from Candida, Aspergillus niger, Aspergillus flavus, and Trichophyton.

## Patentansprüche

1. Verbindung mit einer Struktur, wie in der folgenden Verbindung BAB159 gezeigt:

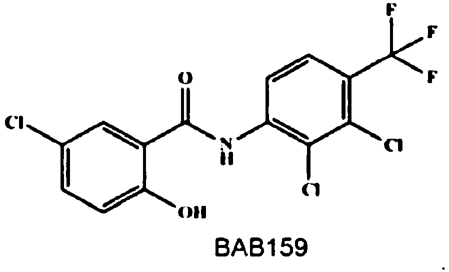

2. Zusammensetzung, umfassend die Verbindung BAB159 nach Anspruch 1 und Polymyxin E als Wirkstoffe;
wobei ein Konzentrationsverhältnis von BAB159 zu Polymyxin E 1-3:1-3 beträgt.

3. Verbindung nach Anspruch 1 oder Zusammensetzung nach Anspruch 2 zur Verwendung bei der antibakteriellen und/oder antimykotischen Behandlung.

4. Verbindung oder Zusammensetzung zur Verwendung nach Anspruch 3, wobei die Bakterien mindestens eines enthalten, das ausgewählt ist aus Staphylococcus, Clostridium perfringens, Enterococcus, Bacillus, Streptococcus, Haemophilus und Mycobacterium smegmatis; und die Pilze mindestens einen enthalten, der ausgewählt ist aus Candida, Aspergillus niger, Aspergillus flavus und Trichophyton.

## Revendications

1. Composé, présentant une structure telle que représentée dans le composé suivant BAB159 :

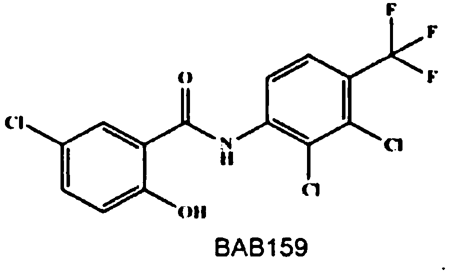

2. Composition, comprenant le composé BAB159 de la revendication 1 et la polymyxine E en tant qu'ingrédients actifs ;
un rapport de concentration de BAB159 à la polymyxine E étant de 1-3:1-3.

3. Composé selon la revendication 1 ou composition selon la revendication 2, destiné(e) à être utilisé(e) dans un traitement antibactérien et/ou antifongique.

4. Composé ou composition destiné(e) à être utilisé(e) selon la revendication 3, lesdites bactéries comprenant au moins l'une choisie parmi Staphylococcus, Clostridium perfringens, Enterococcus, Bacillus, Streptococcus, Haemophilus et Mycobacterium smegmatis ; et lesdits champignons comprenant au moins l'un choisi parmi Candida, Aspergillus niger, Aspergillus flavus et Trichophyton.
